# EUROPEAN PATENT APPLICATION

(11) **EP 1 813 273 A1**
(43) Date of publication of application: **01.08.2007**
(21) Application number: 07105992.7
(22) Date of filing: 08.11.1999
(51) Int. Cl.: A61K 31/353, A61K 31/4439, A61K 9/32, A61K 9/52, A61K 45/06, A61P 3/10

(54) **Pharmaceutical composition for modified release of an insulin sensitiser and another antidiabetic agent**

(30) Priority: 12.11.1998 GB 9824866; 12.11.1998 GB 9824867; 12.11.1998 GB 9824869; 25.05.1999 GB 9912193; 25.05.1999 GB 9912190; 25.05.1999 GB 9912191
(62) Divisional of application: 99964483.4
(71) Applicant: SMITHKLINE BEECHAM PLC, Brentford, Middlesex TW8 9GS (GB)
(72) Inventor: Lewis, Karen, Harlow, Essex CM19 5AW (GB); Lilliott, Nicola Jayne, Harlow, Essex CM19 5AW (GB); Mackenzie, Donald Colin, Harlow, Essex CM19 5AW (GB); Re, Vincenzo, Harlow, Essex CM19 5AW (GB)
(74) Representative: Griffith, Johanna Elise

(57) **Abstract**

A pharmaceutical composition, which composition comprises: an insulin sensitiser and another antidiabetic agent and a pharmaceutically acceptable carrier therefor, wherein the composition is arranged to provide a modified release of at least one of the insulin sensitiser and the other antidiabetes agent, and the use of such composition in medicine.

## Description

This invention relates to a novel composition, in particular to a modified release composition and its use in medicine, especially its use for the treatment of diabetes mellitus, preferably Type 2 diabetes, and conditions associated with diabetes mellitus.

Alpha glucosidase inhibitor antihyperglycaemic agents (or alpha glucosidase inhibitors) and biguanide antihyperglycaemic agents (or biguanides) are commonly used in the treatment of Type 2 diabetes. Acarbose, voglibose, emiglitate and miglitol are examples of alpha glucosidase inhibitors. 1,1 - Dimethylbiguanidine (or metformin) is a particular example of a biguanide.

Insulin secretagogues are compounds that promote increased secretion of insulin by the pancreatic beta cells. The sulphonylureas are well known examples of insulin secretagogues. The sulphonylureas act as hypoglycaemic agents and are used in the treatment of Type 2 diabetes. Examples of sulphonylureas include glibenclamide (or glyburide), glipizide, gliclazide, glimepiride, tolazamide and tolbutamide.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

The above mentioned publications are incorporated herein by reference.

It is now indicated that certain modified release pharmaceutical compositions allow administration of a single daily dose of Compound (I) and another antidiabetic agent, such as an alpha glucosidase inhibitor, a biguanide or an insulin secretagogue, to provide an advantageous delivery of drug for maintaining effective glycaemic control with no observed adverse side effects. Such modified release is therefore considered to be particularly useful for the delivery of insulin sensitisers in combination with other antidiabetic agents for the treatment of diabetes mellitus, especially Type 2 diabetes and conditions associated with diabetes mellitus.

Accordingly, the invention provides a pharmaceutical composition, suitable for the treatment of diabetes mellitus, especially Type 2 diabetes and conditions associated with diabetes mellitus in a mammal, such as a human, which composition comprises: an insulin sensitiser, such as Compound (I), and another antidiabetic agent, such as an alpha glucosidase inhibitor, a biguanide or an insulin secretagogue, and a pharmaceutically acceptable carrier therefor, wherein the composition is arranged to provide a modified release of at least one of the insulin sensitiser and the other antidiabetic agent.

In another aspect, the invention provides a modified release pharmaceutical composition, suitable for the treatment of diabetes mellitus, especially Type 2 diabetes and conditions associated with diabetes mellitus in a mammal, such as a human, which composition comprises: an insulin sensitiser, such as Compound (I), and another antidiabetic agent, such as an alpha glucosidase inhibitor, a biguanide or an insulin secretagogue, and a pharmaceutically acceptable carrier therefor, wherein the carrier is arranged to provide a modified release of at least one of the insulin sensitiser and the other antidiabetic agent

Suitably, the release of both the insulin sensitiser and the other antidiabetic agent is modified.

However, it is envisaged that the release of only the insulin sensitiser is modified. It is also envisaged that the release of only the other antidiabetic agent is modified. The remaining active agent would of course be subject to non-modified release.

Suitably, the modified release is delayed, pulsed or sustained release.

In one aspect the modified release is a delayed release.

Delayed release is conveniently obtained by use of a gastric resistant formulation such as an enteric formulation, such as a tablet coated with a gastric resistant polymer, for example Eudragit L100-55. Other gastric resistant polymers include methacrylates, cellulose acetate phthalate, polyvinyl acetate phthalate, hydroxypropyl methylcellulose phtahlate, in particular, Aquateric, Sureteric, HPMCP-HP-55S.

The enteric coated tablet may be a single layer tablet, where the active agents are admixed prior to compression into tablet form, or a multi-layer tablet, such as a bi-or trilayer tablet, wherein each active agent is present in a discrete layer within the compressed tablet form. The discrete table layers can be arranged as required to provide modified or non-modified release of each active agent.

In a further aspect the modified release is a sustained release, for example providing effective release of active agents over a time period of up to 26 hours, typically in the range of 4 to 24 hours.

Sustained release is typically provided by use of a sustained release matrix, usually in tablet form, such as disintegrating, non-disintegrating or eroding matrices.

Sustained release is suitably obtained by use of a non disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phtahlate, in particular Eudragit L and RL , Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a semi-permeable membrane coated tablet for example by applying methacrylates, ethylcellulose, cellulose acetate, in particular Eudragit RS, Surelease to the tablet.

Sustained release can also be achieved by using a multi layer tablet, where each active ingredient is formulated together or as a separate layer, for example as a matrix tablet, with the other layers providing further control for sustained release of either one or both active agents.

In yet a further aspect the modified release is a pulsed release, for example providing up to 4, for example 2, pulses of active agent per 24 hours.

One form of pulsed release is a combination of non-modified release of active agent and delayed release.

Suitable modified release includes controlled release. The composition of the invention also envisages a combination of pulsed, delayed and/or sustained release for each of the active agents, thereby enabling for example the release of the reagents at different times. For example, where the composition comprises an insulin sensitiser and a biguanide, such as metformin, the composition can be arranged to release the metformin overnight.

A suitable alpha glucosidase inhibitor is acarbose.

Other suitable alpha glucosidase inhibitors are emiglitate and miglitol. A further suitable alpha glucosidase inhibitor is voglibose.

Suitable biguanides include metformin, buformin or phenformin, especially metformin.

Suitable insulin secretagogues include sulphonylureas.

Suitable sulphonylureas include glibenclamide, glipizide, gliclazide, glimepiride, tolazamide and tolbutamide. Further sulphonylureas include acetohexamide, carbutamide, chlorpropamide, glibornuride, gliquidone, glisentide, glisolamide, glisoxepide, glyclopyamide and glycylamide. Also included is the sulphonylurea glipentide.

Further suitable insulin secretagogues include repaglinide. An additional insulin secretagogue is nateglinide.

A preferred thiazolidinedione insulin sensitiser is Compound (I).

Other suitable thiazolidinedione insulin sensitisers include (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

A particular thiazolidinedione insulin sensitiser is 5-[4-[2-(5-ethylpyridin-2-yl)ethoxy]benzyl] thiazolidine-2,4-dione (or pioglitazone).

A particular thiazolidinedione insulin sensitiser is (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone).

Suitable dosages, preferably unit dosages, of the insulin sensitiser and the other antidiabetic agent, such as the alpha glucosidase inhibitor, a biguanide or insulin secretagogue, include the known permissible doses for these compounds as described or referred to in reference texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or the above mentioned publications.

The dosages of each particular active agent in any given composition can as required vary within a range of doses known to be required in respect of accepted dosage regimens for that compound. Dosages of each active agent can also be adapted as required to take into account advantageous effects of combining the agents as mentioned herein.

In one particular aspect, the composition comprises 2 to 12 mg of Compound (I).

Suitably the composition comprises 2, 3, 4, 5, 6, 7, 8, 9, 10, 11 or 12 mg of Compound (I).

Particularly, the composition comprises 2 to 4 , 4 to 8 or 8 to 12 mg of Compound (I).

Particularly, the composition comprises 2 to 4mg of Compound (I).

Particularly, the composition comprises 4 to 8mg of Compound (I).

Particularly, the composition comprises 8 to 12 mg of Compound (I).

Preferably, the composition comprises 2 mg of Compound (I).

Preferably, the composition comprises 4 mg of Compound (I).

Preferably, the composition comprises 8 mg of Compound (I).

Suitable unit dosages of other insulin sensitisers include from 100 to 800mg of troglitazone such as 200, 400, 600 or 800mg or from 5 to 50mg, including 10 to 40mg, of pioglitazone, such as 20, 30 or 40 mg and also including 15, 30 and 45mg of pioglitazone.

As indicated above the unit doses of the additional antidiabetic agents including the alpha glucosidase inhibitor, the biguanide and the insulin secretagogue include those found in the reference texts mentioned herein and include the doses set out below.

For the alpha glucosidase inhibitor, a suitable amount of acarbose is in the range of from 25 to 600 mg, including 50 to 600 mg, for example 100mg or 200mg.

For the the biguanide, a suitable dosage of metformin is between 100 to 3000mg, for example 250, 500mg, 850mg or 1000mg.

For the insulin secretagogue, a suitable amount of glibenclamide is in the range of from 2.5 to 20 mg, for example 10mg or 20mg; a suitable amount of glipizide is in the range of from 2.5 to 40 mg; a suitable amount of gliclazide is in the range of from 40 to 320 mg; a suitable amount of tolazamide is in the range of from 100 to 1000 mg; a suitable amount of tolbutamide is in the range of from 1000 to 3000 mg; a suitable amount of chlorpropamide is in the range of from 100 to 500 mg; and a suitable amount of gliquidone is in the range of from 15 to 180 mg. Also a suitable amount of glimepiride is 1 to 6mg and a suitable amount of glipentide is 2.5 to 20mg.

A suitable amount of repaglinide is in the range of from 0.5mg to 20mg, for example 16mg. Also a suitable amount of nateglinide is 90 to 360mg, for example 270mg.

The compounds mentioned herein, in particular the thiazolidinediones such as Compound (I), may exist in one of several tautomeric forms, all of which are encompassed by the invention as individual tautomeric forms or as mixtures thereof. The compounds mentioned herein may contain one or more chiral carbon atoms and hence can exist in two or more stereoisomeric forms, all of which are encompassed by the invention either as individual isomers or as mixtures of isomers, including racemates.

It will be understood that the insulin sensitiser, such as Compound (I) and the other antidiabetic agent are in a pharmaceutically acceptable form, including pharmaceutically acceptable derivatives such as pharmaceutically acceptable salts, esters and solvates thereof, as appropriate to the relevant pharmaceutically active agent chosen. In certain instances herein the names used for the antidiabetic agent may relate to a particular pharmaceutical form of the relevant active agent: It will be understood that all pharmaceutically acceptable forms of the active agents per se are encompassed by this invention.

Suitable pharmaceutically acceptable forms of the insulin sensitiser and other antidiabetic agent depend upon the particular agent used but included are known pharmaceutically acceptable forms of the particular agent chosen. Such derivatives are found or are referred to in standard reference texts such as the British and US Pharmacopoeias, Remington's Pharmaceutical Sciences (Mack Publishing Co.), The Extra Pharmacopoeia (London, The Pharmaceutical Press) (for example see the 31st Edition page 341 and pages cited therein) and the above mentioned publications. For example, a particular form of metformin is metformin hydrochloride, a particular form of repaglinide is a benzoic acid salt form and a particular form of tolbutamide is a sodium salt form.

Suitable pharmaceutically acceptable forms of Compound (I) include those described in EP 0306228 and WO94/05659, especially pharmaceutically acceptable salted or solvated forms. A preferred pharmaceutically acceptable salt form of Compound (I) is a maleate. A preferred pharmaceutically acceptable solvated form of Compound (I) is a hydrate. A preferred form of pioglitazone is as the hydrochloride salt.

The insulin sensitiser or the alpha glucosidase inhibitor antihyperglycaemic agent of choice is prepared according to known methods, such methods are found or are referred to in standard reference texts, such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or as described in the above mentioned publications.

Compound (I) or, a pharmaceutically acceptable salt thereof, or a pharmaceutically acceptable solvate thereof, may be prepared using known methods, for example those disclosed in EP 0306228 and WO94/05659. The disclosures of EP 0306228 and WO94/05659 are incorporated herein by reference.

When used herein the term 'conditions associated with diabetes' includes those conditions associated with the pre-diabetic state, conditions associated with diabetes mellitus itself and complications associated with diabetes mellitus.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy.

Renal diseases associated with Type 2 diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

For the avoidance of doubt, unless other wise stated, when reference is made herein to scalar amounts, including mg amounts, of the active compound such as Compound (I), in a pharmaceutically acceptable form, the scalar amount referred to is made in respect of the active compound *per se:* For example 2 mg of Compound (I) in the form of the maleate salt is that amount of maleate salt which provides 2 mg of Compound (I).

Diabetes mellitus is preferably Type 2 diabetes.

Glycaemic control may be characterised using conventional methods, for example by measurement of a typically used index of glycaemic control such as fasting plasma glucose or glycosylated haemoglobin (Hb A1c). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

In a preferred aspect, the dosage level of each of the active agents when used in accordance with the treatment of the invention will be less than would have been required from a purely additive effect upon glycaemic control.

There is also an indication that the treatment of the invention will effect an improvement, relative to the non-modified release of the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

Usually the compositions are adapted for oral administration. However, they may be adapted for other modes of administration, for example parenteral administration, sublingual or transdermal administration.

In a further aspect the invention also provides a process for preparing a pharmaceutical composition, suitably for the treatment of diabetes mellitus, especially Type 2 diabetes and conditions associated with diabetes mellitus in a mammal, such as a human, which composition comprises an insulin sensitiser, such as Compound (I), and another antidiabetic agent, such as an alpha glucosidase inhibitor, a biguanide or an insulin secretagogue, and a pharmaceutically acceptable carrier therefor, which process comprises formulating the insulin sensitiser, the other antidiabetic agent and the pharmaceutically acceptable carrier so as to enable a modified release of at least one of the insulin sensitiser and the other antidiabetic agent.

In a further aspect, the invention provides a process for preparing a modified release pharmaceutical composition, suitably for the treatment of diabetes mellitus, especially Type 2 diabetes and conditions associated with diabetes mellitus in a mammal, such as a human, which composition comprises an insulin sensitiser, such as Compound (I) and another antidiabetic agent, such as an alpha glucosidase inhibitor, a biguanide or an insulin secretagogue and a pharmaceutically acceptable carrier therefor, which process comprises formulating the insulin sensitiser, the other antidiabetic agent and the pharmaceutically acceptable carrier so as to enable a modified release of at least one of the insulin sensitiser and the other antidiabetic agent.

The compositions are formulated to provide the modified release of active agents according to the appropriate methods required, for example those disclosed in Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) and Harry's Cosmeticology (Leonard Hill Books).

Preferably, the compositions are in unit dosage form. Unit dosage presentation forms for oral administration may be in tablet or capsule form and may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

As required the solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice.

Compositions may, if desired, be in the form of a pack accompanied by written or printed instructions for use.

No adverse toxicological effects are expected for the compositions of the invention in the above mentioned dosage ranges.

### EXAMPLES COMPRISING AN INSULIN SENSITISER AND A BIGUANIDE

### Example 1, Delayed Release Composition

Delayed release is achieved by coating single or bilayer tablets comprising 4mg or 8mg of Compound (I) as pure free base (pfb) and 500, preferably, or 1000 or 1500mg of metformin HCl with Eudragit L100-55, a gastric resistant polymer

The enteric coat consists of:

| | %w/w |
|---|---|
| Eudragit L30 D-55 (30% aqueous dispersion) | 76.8 |
| Triethyl Citrate | 7.7 |
| Talc Alphafil 500 | 15.5 |

### Example 2, Sustained release by use of a semi-permeable membrane

The semi-permeable membrane consists of:

| | %w/w |
|---|---|
| Eudragit RS30D (30% aqueous dispersion) | 90 |
| Triethyl Citrate | 1 |
| Talc | 9 |

This membrane is applied to a single or bilayer tablets each comprising 4mg or 8mg of Compound (I) and 500, preferably, or 1000 or 1500mg of metformin HCl

### Example 3, Sustained Release by use of a non disintegrating matrix tablet

A matrix tablet is formed by tabletting the following mixture as:
(a) a single layer tablet:

| | mg/tablet |
|---|---|
| Compound (I) | 4 (pfb) |
| Metformin HCl | 500 |
| Eudragit L100-55 | 150 |
| Lactose monohydrate | 50 |
| Eudragit RS powder to | 1000 |

(b) a bilayer tablet to provide sustained release of Compound I and immediate (i.e non-modified) release of metformin HCl.

| Layer A | mg/tablet |
|---|---|
| Compound (I) | 4 (pfb) |
| Eudragit L100-55 | 150 |
| Lactose monohydrate | 50 |
| Eudragit RS powder to | 500 |

| Layer B | mg/tablet |
|---|---|
| Metformin HCl | 500 |
| Polyvinyl pyrollidone | 15 |
| Magnesium stearate to | 520 |

### Example 4, Sustained Release by use of a Mixed Eudragit matrix tablet

A matrix tablet is formed by tabletting the following mixture as:
(a) a single layer tablet:

| | mg/tablet |
|---|---|
| Compound (I) | 4 (pfb) |
| Metformin HCl | 500 |
| Eudragit L100-55 | 74 |
| Eudragit RS powder | 18.5 |
| Colloidal Silicon dioxide | 2.6 |
| Magnesium stearate | 3.25 |
| Lactose monohydrate to | 650 |

(b) a trilayer tablet:

| | |
|---|---|
| Layer A | mg/tablet |
| Compound (I) | 4 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

| Layer B | mg/tablet |
|---|---|
| Metformin HCl | 250 |
| Eudragit L100-55 | 74 |
| Eudragit RS powder to | 345 |

| Layer C | mg/tablet |
|---|---|
| Metformin HCl | 250 |
| Polyvinyl pyrrolidone | 7.5 |
| Magnesium stearate to | 260 |

### Example 5, Sustained Release by use of a disintegrating matrix tablet

A matrix tablet is formed by tabletting the following mixture as a single layer tablet:

| | mg/tablet |
|---|---|
| Compound (I) | 4 (pfb) |
| Metformin HCl | 500 |
| Eudragit L100-55 | 74 |
| Methocel K4M | 18.5 |
| Colloidal Silicon dioxide | 2.6 |
| Magnesium stearate | 3.25 |
| Lactose monohydrate to | 650 |

### Example 6, Sustained Release by use of a Mixed Carbopol matrix tablet

A matrix tablet is formed by tabletting the following mixture as single or bilayer tablet:

| | mg/tablet |
|---|---|
| Compound (I) | 4 (pfb) |
| Metformin HCl | 500 |
| Anhydrous dibasic calcium phosphate | 35.7 |
| Carbopol 971P | 22.5 |
| Carbopol 974P | 7.5 |
| Talc | 0.75 |
| Lactose monohydrate to | 650 |

### Example 7, Delayed Release Composition

A capsule containing multiple pellet cores is formed using the following mixture:

| | mg/capsule |
|---|---|
| Compound (I) | 4 (pfb) |
| Metformin HCl | 500 |
| Microcrystalline cellulose to | 650 |

Delayed release can be achieved by coating the pellet cores with Eudragit L100-55, a gastric resistant polymer as in example 1.

### EXAMPLES COMPRISING AN INSULIN SENSITISER AND AN INSULIN SECRETAGOGUE

### Example 1, Delayed Release Composition

Delayed release can be achieved by coating single or bilayer tablets comprising 4mg or 8mg of Compound (I) as pure free base (pfb) and 2.5, 10 or 20 mg of glibenclamide with Eudragit L100-55, a gastric resistant polymer

The enteric coat consists of:

| | %w/w |
|---|---|
| Eudragit L30 D-55 (30% aqueous dispersion) | 76.8 |
| Triethyl Citrate | 7.7 |
| Talc Alphafil 500 | 15.5 |

### Example 2, Sustained Release by use of a matrix tablet (single layer)

A matrix tablet is formed by tabletting the following mixture as a single layer tablet:

| | mg/tablet |
|---|---|
| Compound (I) | 8 (pfb) |
| glibenclamide | 10 |
| Eudragit L100-55 | 150 |
| Lactose monohydrate | 50 |
| Eudragit RS powder to | 500 |

### Example 3, Sustained Release and Non-modifie Release by use of a matrix tablet (bilayer)

A matrix tablet is formed by tabletting the following mixture as a bilayer tablet to provide sustained release of Compound I and immediate (i.e non-modified) release of glibenclamide :

| Layer A | mg/tablet |
|---|---|
| Compound (I) | 8 (pfb) |
| Eudragit L100-55 | 150 |
| Lactose monohydrate | 50 |
| Eudragit RS powder to: | 500 |

| Layer B | mg/tablet |
|---|---|
| Glibenclamide | 10 |
| Polyvinylpyrrolidone | 12.5 |
| Sodium starch glycolate | 10 |
| Lactose monhydrate to | 250 |

### Example 4, Sustained release by use of a semi-permeable membrane

The semi-permeable membrane consists of:

| | %w/w |
|---|---|
| Eudragit RS30D (30% aqueous dispersion) | 90 |
| Triethyl Citrate | 1 |
| Talc | 9 |

This membrane is applied to a single or multi layer tablet each comprising 4mg or 8mg Compound (I) (pfb) and 2.5, 10 (preferably) or 20mg Glibenclamide.

### Example 5, Sustained Release by use of a Mixed Eudragit matrix tablet

A matrix tablet is formed by tabletting the following mixture as:
(a) a single layer tablet:

| | mg/tablet |
|---|---|
| Compound (I) | 8 (pfb) |
| Glibenclamide | 10 |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

(b) a bilayer tablet:

| Layer A | mg/tablet |
|---|---|
| Compound (I) | 8 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

| Layer B | mg/tablet |
|---|---|
| Glibenclamide | 10 |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

### Example 6, Sustained Release by use of a Mixed Carbopol matrix tablet

A matrix tablet is formed by tabletting the following mixture as single or bilayer tablet:

| | mg/tablet |
|---|---|
| Compound (I) | 8 (pfb) |
| Glibenclamide | 10 |
| Anhydrous dibasic calcium phosphate | 35.7 |
| Carbopol 971P | 22.5 |
| Carbopol 974P | 7.5 |
| Talc | 0.75 |
| Lactose monohydrate to | 150 |

### Example 7, Delayed Release Composition

A capsule containing multiple pellet cores is formed using the following mixture:

| | mg/capsule |
|---|---|
| Compound (I) | 8 (pfb) |
| Glibenclamide | 10 |
| Microcrystalline cellulose | 133.5 |
| Lactose monohydrate to | 267 |

Delayed release can be achieved by coating the pellet cores with Eudragit L100-55, a gastric resistant polymer as in example 1.

### EXAMPLES COMPRISING AN INSULIN SENSITISER AND AN ALPHA GLUCOSIDASE INHIBITOR.

### Example 1, Delayed Release Composition

Delayed release can be achieved by coating single or bilayer tablets comprising 4mg or 8mg of Compound (I) as pure free base (pfb) and 100mg acarbose with Eudragit L100-55, a gastric resistant polymer

The enteric coat consists of:

| | %w/w |
|---|---|
| Eudragit L30 D-55 (30% aqueous dispersion) | 76.8 |
| Triethyl Citrate | 7.7 |
| Talc Alphafil 500 | 15.5 |

### Example 2, Sustained Release by use of a matrix tablet

A matrix tablet is formed by tabletting the following mixture as:
(a) a single layer tablet:

| | mg/tablet |
|---|---|
| Compound (I) | 8 (pfb) |
| Acarbose | 100 |
| Eudragit L100-55 | 150 |
| Lactose monohydrate | 50 |
| Eudragit RS powder to | 600 |

(b) a bilayer tablet to provide sustained release of Compound (I) and non modified (i.e immediate) release of acarbose :

| Layer A | mg/tablet |
|---|---|
| Compound (I) | 8 (pfb) |
| Eudragit L100-55 | 150 |
| Lactose monohydrate | 50 |
| Eudragit RS powder to | 500 |

| Layer B | mg/tablet |
|---|---|
| Acarbose | 100 |
| Microcrystalline cellulose | 134 |
| Starch | 12.5 |
| Colloidal silicon dioxide | 1.25 |
| Magnesium stearate to | 250 |

### Example 3, Sustained release by use of a semi-permeable membrane

The semi-permeable membrane consists of:

| | %w/w |
|---|---|
| Eudragit RS30D (30% aqueous dispersion) | 90 |
| Triethyl Citrate | 1 |
| Talc | 9 |

This membrane is applied to a single or multi layer tablets each comprising 4mg or 8mg Compound (I) (pfb) and 100mg Acarbose

### Example 4, Sustained Release by use of a Mixed Eudragit matrix tablet

A matrix tablet is formed by tabletting the following mixture as:
(a) a single layer tablet:

| | mg/tablet |
|---|---|
| Compound (I) | 8 (pfb) |
| Acarbose | 100 |
| Eudragit L100-55 | 74 |
| Eudragit RS powder | 18.5 |
| Colloidal Silicon dioxide | 1 |
| Magnesium stearate | 2.5 |
| Lactose monohydrate to | 250 |

(b) a bilayer tablet:

| Layer A | mg/tablet |
|---|---|
| Compound (I) | 8 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate to | 150 |

| Layer B | mg/tablet |
|---|---|
| Acarbose | 100 |
| Eudragit L100-55 | 74 |
| Eudragit RS powder | 18.5 |
| Colloidal Silicon dioxide | 0.6 |
| Magnesium stearate | 1.5 |
| Lactose monohydrate to | 250 |

### Example 5, Sustained Release by use of a Disintegrating matrix tablet

A matrix tablet is formed by tabletting the following mixture as a single layer tablet:

| | mg/tablet |
|---|---|
| Compound (I) | 8 (pfb) |
| Acarbose | 100 |
| Eudragit L100-55 | 74 |
| Methocel K4M | 18.5 |
| Colloidal Silicon dioxide | 1 |
| Magnesium stearate | 2.5 |
| Lactose monohydrate to | 250 |

### Example 6, Sustained Release by use of a Mixed Carbopol matrix tablet

A matrix tablet is formed by tabletting the following mixture as a single or bilayer tablet:

| | mg/tablet |
|---|---|
| Compound (I) | 8 (pfb) |
| Acarbose | 100 |
| Anhydrous dibasic calcium phosphate | 35.7 |
| Carbopol 971P | 22.5 |
| Carbopol 974P | 7.5 |
| Talc | 0.75 |
| Lactose monohydrate to | 250 |

### Example 7, Delayed Release Composition

A capsule containing multiple pellet cores is formed using the following mixture:

| | mg/capsule |
|---|---|
| Compound (I) | 8 (pfb) |
| Acarbose | 100 |
| Microcrystalline cellulose | 133.5 |
| Lactose monohydrate to | 267 |

Delayed release can be achieved by coating the pellet cores with Eudragit L100-55, a gastric resistant polymer as in example 1.

## Claims

**1.** A pharmaceutical composition, which composition comprises: an insulin sensitiser and another antidiabetic agent and a pharmaceutically acceptable carrier therefor, wherein the composition is arranged to provide a modified release of at least one of the insulin sensitiser and the other antidiabetic agent.

**2.** A modified release pharmaceutical composition, which composition comprises: an insulin sensitiser, such as Compound (I), and another antidiabetic agent and a pharmaceutically acceptable carrier therefor, wherein the carrier is arranged to provide a modified release of at least one of the insulin sensitiser and the other antidiabetes agent.

**3.** A composition according to claim 1 or claim 2, wherein the release of both the insulin sensitiser and the other antidiabetes agent is modified.

**4.** A composition according to any one of claims 1 to 3, wherein the modified release is a delayed release.

**5.** A composition according to claim 4, wherein the composition is in the form of an enteric tablet formulation.

**6.** A composition according to claim 5, wherein the enteric coated tablet is a single layer tablet.

**7.** A composition according to claim 7, wherein the enteric coated tablet is a multi-layer tablet.

**8.** A composition according to any one of claims 5 to 7, wherein the tablet is coated with a gastric resistant polymer.

**9.** A composition according to claim 8, wherein the gastric resistant polymer is selected from the list consisting of Eudragit L100-55, methacrylates, cellulose acetate phthalate, polyvinyl acetate phthalate, hydroxypropyl methylcellulose phtahlate, in particular, Aquateric, Sureteric and HPMCP-HP-55S.

**10.** A composition according to any one of claims 1 to 3, wherein the modified release is a sustained release.

**11.** A composition according to any one of claims 1 to 3, wherein the sustained release is provided by a sustained release matrix selected from disintegrating, non-disintegrating and eroding matrices.

**13.** A composition according to claim 11, wherein the non disintegrating matrix tablet formulation is provided by incorporating Eudragit RS, methacrylates, cellulose acetates, hydroxypropyl methylcellulose phthalate, Carbopol 971P or HPMCP-HP-55S into the matrix.

**14.** A composition according to claim 11, wherein the disintegrating matrix tablet formulation is provided by incorporating methacrylates, methylcellulose and Methocel K4M into the matrix.

**15.** A composition according to any one of claims 1 to 14, wherein the insulin sensitiser is 5-[4-[2-(N-methyl-N-(2-pyridyl)amino)ethoxy]benzyl]thiazolidine-2,4-dione, 5-[4-[2-(5-ethylpyridin-2-yl)ethoxy]benzyl] thiazolidine-2,4-dione (pioglitazone) or (+) -5-[[4-[(3,4-dihydro-6-hydroxy-2, 5, 7, 8-tetramethyl-2H-1-benzopyran-2-yl)methoxy]phenyl]methyl]-2,4-thiazolidinedione (troglitazone); or a derivative thereof.

**16.** A composition according to any one of claims 1 to 15, wherein the alpha glucosidase inhibitor is acarbose, emiglitate, miglitol or voglibose.

**17.** A composition according to any one of claims 1 to 15, wherein the biguanide is metformin, buformin or phenformin.

**18.** A composition according to any one of claims 1 to 15, wherein the insulin secretagogues is a sulphonylurea selected from glibenclamide, glipizide, gliclazide, glimepiride, tolazamide, tolbutamide, acetohexamide, carbutamide, chlorpropamide, glibornuride, gliquidone, glisentide, glisolamide, glisoxepide, glyclopyamide and glycylamide, glipentide.

**19.** A composition according to any one of claims 1 to 15, wherein the insulin secretagogue is repaglinide or nateglinide.
